(19) Europäisches Patentamt
European Patent Office
Office européen des brevets

(11) Veröffentlichungsnummer: **0 066 212**
**B1**

(12) # EUROPÄISCHE PATENTSCHRIFT

(45) Veröffentlichungstag der Patentschrift: **24.10.84**

(51) Int. Cl.³: **C 07 C 85/24,** C 07 C 87/40

(21) Anmeldenummer: **82104443.5**

(22) Anmeldetag: **21.05.82**

(54) **Verfahren zur katalytischen Hydrierung von Di-(4-aminophenyl)methan.**

(30) Priorität: **01.06.81 US 269199**

(43) Veröffentlichungstag der Anmeldung: **08.12.82 Patentblatt 82/49**

(45) Bekanntmachung des Hinweises auf die Patenterteilung: **24.10.84 Patentblatt 84/43**

(84) Benannte Vertragsstaaten: **BE DE FR GB IT NL**

(56) Entgegenhaltungen:
**DE - A - 1 948 566**
**DE - A - 2 423 639**
**FR - A - 2 372 142**
**GB - A - 1 122 609**

**CHEMICAL ABSTRACTS, Band 77, Nr. 25, 18. Dezember 1972, Seite 368, Nr. 164129g, Columbus Ohio (USA)**

(73) Patentinhaber: **Mobay Chemical Corporation, Penn Lincoln Parkway West, Pittsburgh, Pennsylvania 15205 (US)**

(72) Erfinder: **Allen, Gary F., 811 Clearview Terrace, New Martinsville WV 26155 (US)**

(74) Vertreter: **Drope, Rüdiger, Dr. et al, c/o Bayer AG Zentralbereich Patente Marken und Lizenzen, D-5090 Leverkusen 1, Bayerwerk (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 066 212 B1**

ACTORUM AG

**Beschreibung**

Bei der Herstellung von Di-(4-aminocyclohexyl)methan durch katalytische Hydrierung von Di-(4-aminophenyl)methan werden im wesentlichen drei Stereoisomere gebildet:

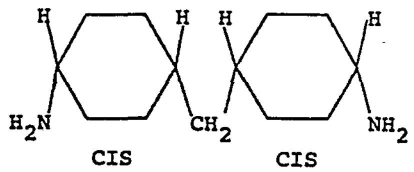

CIS CIS

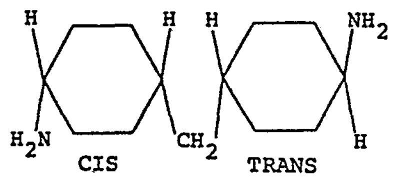

CIS TRANS

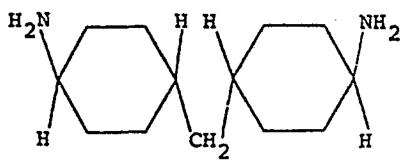

TRANS TRANS

Es ist in der Fachwelt bekannt, dass für die Herstellung eines entsprechenden Isocyanats (mittels des bekannten Verfahrens der Phosgenierung), das bei Raumtemperatur (d.h. von 20 bis 25°C) flüssig und lagerbeständig ist, die Mischung der für die Phosgenierung eingesetzten Aminstereoisomeren das Trans,transstereoisomere nur in relativ kleineren Mengen (typischerweise von 15 bis 40, vorzugsweise von 18,5 bis 23,5 Gew.-%) enthalten darf.

In der Fachwelt sind zahlreiche Verfahrensweisen zur Herstellung solcher Amingemische bekannt, die die geforderte Menge des Trans,transisomeren enthalten. Repräsentativ für diese Verfahrensweisen sind diejenigen, die in den US-PS Nrn. 3153088, 3155724, 3393236, 3644522, 3711550 und 3766272 beschrieben sind. Diese bekannten Verfahren erfordern im allgemeinen die Abtrennung eines die geforderte Menge des Trans, transisomeren enthaltenden Amingemisches aus einem Amingemisch, das durch die Hydrierung gebildet wird und etwa 50 Gew.-% des Trans, transisomeren enthält. In der Fachwelt sind auch Verfahren zur direkten Herstellung eines die geforderte Menge des Trans,transisomeren enthaltenden Amingemisches aus Di-(4-aminophenyl)-methan bekannt, die die Zwischenstufe der Abtrennung entbehrlich machen (vgl. die US-PS Nr. 2606928). Jedoch sind hierbei die Reaktionsgeschwindigkeiten für Zwecke technischer Anwendungen viel zu niedrig.

Die gebräuchlichste katalytische Hydrierung von Di-(4-aminophenyl)methan zu Di-(4-aminocyclohexyl)methan verwendet Rutheniumkatalysatoren in Form von Trägerkatalysatoren oder trägerfreien Katalysatoren. Typische Verfahren dieser Art sind in den US-PS Nrn. 2494563, 2606924, 2606928, 2606925, 3347917, 3676495, 3959374, 3743677, 3914307, 3825586, 3636108 und 4161492 beschrieben. Wenngleich einige dieser Verfahren ein Amingemisch liefern, das das Trans,transisomere in einer Menge enthält, die die Herstellung flüssiger, lagerbeständiger Isocyanate ermöglicht, sind die Reaktionsgeschwindigkeiten viel zu niedrig für einen technischen Einsatz.

Die Verwendung eines Rhodiumträgerkatalysators bei der Hydrierung von Di-(4-aminophenyl)-methan ist ebenfalls bekannt (vgl. die US-PS Nrn. 3591635 und 3856862 bzw. die entsprechenden DE-OS Nrn. 1948566 und 2423639). Diese Verfahren ergeben bei relativ milden Reaktionsbedingungen mit grossen Mengen an Katalysator und langen Reaktionszeiten feste Hydrierprodukte, die für die Weiterverarbeitung (z.B. Phosgenierung) nicht gut geeignet sind. Die gleichzeitige Anwendung relativ hoher Drucke und Temperaturen wird dabei ausdrücklich ausgeschlossen.

Die vorliegende Erfindung betrifft ein Verfahren zur Herstellung flüssigen und 15 bis 40, vorzugsweise 18,5 bis 23,5 Gew.-%, des Trans,transisomeren enthaltenden Di-(4-aminocyclohexyl)-methans aus Di-(4-aminophenyl)methan unter Verwendung eines Rhodiumträgerkatalysators und Einhaltung spezieller Verfahrensbedingungen. Die erforderlichen speziellen Verfahrensbedingungen sind ein Wasserstoffdruck von 137,9 bis 551,6 bar (2000 bis 8000 psi), wobei 137,9 bar bis 275,8 bar (2000 bis 4000 psi) besonders bevorzugt werden, und eine während der Hydrierung im Bereich von 196 bis 235, vorzugsweise von 200 bis 225°C, gehaltene Temperatur, wobei der Bereich von 205 bis 219°C besonders bevorzugt wird.

Der bei dem Verfahren gemäss der vorliegenden Erfindung verwendete Katalysator enthält Rhodium auf einem Träger, wie sie üblicherweise für Zwecke der Herstellung von Hydrierkatalysatoren verwendet werden. Beispiele für solche Träger sind Aluminiumoxid, Kohlenstoff, Kieselgur, Bentonit, Asbest, Silicagel, Zirconiumoxid und dergleichen. Der bevorzugt bei dem Verfahren gemäss der vorliegenden Erfindung eingesetzte Träger ist Aluminiumoxid. Die Menge an elementarem Rhodium, die in dem erfindungsgemäss verwendeten Katalysator vorhanden ist, kann beispielsweise etwa von 0,5 bis 20 Gew.-% variiert werden, liegt jedoch vorzugsweise innerhalb des Bereichs von etwa 1 bis 10 Gew.-%. Besonders bevorzugt wird eine in dem Katalysator vorhandene Menge an elementarem Rhodium innerhalb des Bereichs von etwa 1 bis 5 Gew.-%.

Der bei dem erfindungsgemässen Verfahren verwendete Rhodiumträgerkatalysator kann mittels in der Fachwelt wohlbekannter Verfahrensweisen hergestellt werden. Beispielsweise wird eine Suspension des gewünschten Trägers in einer wässerigen Lösung eines löslichen Rhodiumsalzes wie Rhodiumchloridtrihydrat mit einer Base behandelt, um das Rhodiumhydroxid auf dem Träger niederzuschlagen. Wenn dieser in einer Wasserstoffatmosphäre geschüttelt wird, wird das Rhodiumsalz auf dem Träger zur Stufe des Elements reduziert. Die Herstellung des Katalysators auf diese Weise kann in einem getrennten Arbeitsgang durchgeführt werden, oder aber sie kann als Vorstufe in das Verfahren gemäss der Erfindung einbezogen werden. Beispielsweise kann der Katalysator in der vorbeschriebenen Weise in dem Hydriergefäss hergestellt werden, das bei dem erfindungsgemässen Verfahren verwendet werden soll, und der betreffende Katalysator muss dann vor dem Zusatz der zu hydrierenden Charge lediglich mit Wasser gewaschen werden.

Da andererseits viele der bei dem erfindungsgemässen Verfahren verwendeten Rhodiumträgerkatalysatoren in fertig präparierter Form im Handel leicht erhältlich sind, kann auf die praktische Herstellung des Katalysators in einer Vorstufe des Verfahrens auch verzichtet werden, falls dieses erwünscht ist. Tatsächlich ist der zur Zeit bevorzugte Katalysator ein käuflicher Katalysator, der von der Firma Engelhard bezogen werden kann (5% Rhodium auf einem Aluminiumoxidträger). Ebenfalls bevorzugt werden Rhodiumträgerkatalysatoren, die bei der Firma Johnson-Matthey erhältlich sind.

Bei der Durchführung des Verfahrens gemäss der vorliegenden Erfindung gelangen die allgemein in der Fachwelt angewandten Verfahrensweisen zum Einsatz, wobei die einzigen besonderen Anforderungen in den im Vorstehenden angegebenen Bedingungen für Druck und Temperatur bestehen. Die Hydrierung wird vorzugsweise in Gegenwart eines inerten Lösungsmittels durchgeführt, d.h. eines solchen Lösungsmittels, das den angestrebten Verlauf der Hydrierung praktisch nicht stört.

Geeignete Lösungsmittel sind Ether wie Butylether oder Isopropylether, Cyclohexan oder andere aliphatische Kohlenwasserstoffe, Alkohole wie Butylalkohol, Methanol, Ethanol, Isopropanol und Propanol, sowie cyclische Ether wie Tetrahydrofuran und Dioxan. Die Menge des eingesetzten Lösungsmittels kann von 0 bis 95 Gew.-%, bezogen auf das Gesamtgewicht von Amin und Lösungsmittel, betragen. Vorzugsweise wird die Menge des verwendeten Lösungsmittels so bemessen, dass die Konzentration des Ausgangsdiamins in der Reaktionsmischung etwa 5 bis 50 Gew.-% beträgt. Das gegenwärtig bevorzugte Lösungsmittel ist n-Butylether.

Der Katalysator wird in einer Lösung des Ausgangsdiamins suspendiert, und die entstandene Suspension wird in einem geeigneten Hydriergefäss der Hydrierung unterworfen. Es wird eine solche Katalysatormenge verwendet, dass die Menge des in der Reaktionsmischung vorhandenen Rhodiums vorzugsweise mindestens 0,005 Gew.-%, bezogen auf das Gewicht des Ausgangsdiamins, beträgt. Besonders bevorzugt wird ein Rhodiumgehalt im Bereich von etwa 0,01 bis 3 Gew.-%, bezogen auf das in der Reaktionsmischung vorhandene Ausgangsdiamin, und ganz besonders bevorzugt wird eine Katalysatormenge, bei der die Menge des in der Reaktionsmischung vorhandenen Rhodiums etwa innerhalb des Bereichs von 0,02 bis 2 Gew.-%, bezogen auf das eingesetzte Ausgangsdiamin, liegt. Die einzusetzende Höchstmenge an Katalysator wird im allgemeinen durch die Kosten für den Katalysator bestimmt.

Die Hydrierung wird durchgeführt bei einer Temperatur innerhalb des Bereichs von 196 bis 235, vorzugsweise von etwa 200 bis 225°C, wobei der Bereich von etwa 205 bis etwa 219°C besonders bevorzugt wird. Die genaue Wahl der Temperatur in einem bestimmten Falle hängt von der gewünschten Reaktionsgeschwindigkeit und dem angestrebten Gehalt an dem Trans,transisomeren ab. Im allgemeinen läuft die Reaktion um so schneller ab und ist der Trans,trans-Gehalt des Endprodukts um so höher, je höher die Temperatur ist. Die Temperatur wird demnach im allgemeinen so gewählt, dass sie einen optimalen Kompromiss zwischen der Reaktionsdauer und dem Gehalt des Trans,transisomeren ergibt.

Der bei dem Verfahren gemäss der vorliegenden Erfindung angewandte Wasserstoffdruck liegt im Bereich zwischen 137,9 und 551,6 bar (2000 bis 8000 psi). Naturgemäss hängen die Drucke von den verwendeten Apparaturen ab. Im allgemeinen wurde gefunden, dass die Ausbeute mit steigendem Druck zunimmt. Es wurde jedoch auch gefunden, dass der Anteil des Trans,transisomeren in dem Produkt mit steigendem Druck abnimmt.

Das Fortschreiten der Reaktion wird in einfacher Weise durch Beobachtung der von der Reaktionsmischung aufgenommenen Wasserstoff-Menge verfolgt, und die Hydrierung wird zu dem Zeitpunkt beendet, bei dem die theoretische Menge Wasserstoff absorbiert worden ist. Der Katalysator wird dann von der Lösung des reduzierten Materials abgetrennt, und letztere wird destilliert, um das Di-(4-aminocyclohexyl)methan daraus zu isolieren. Die Hydrierzeiten betragen im allgemeinen etwa 10 bis 60 min.

Falls erwünscht, kann auch Ammoniak verwendet werden, wie in den US-PS Nrn. 3591635 und 3856862 beschrieben wird, obwohl dies zur Erzielung der Ergebnisse der vorliegenden Erfindung nicht erforderlich ist.

Im allgemeinen werden die Materialien miteinander vermischt und chargenweise dem Reaktionsgefäss zugeführt, jedoch könnte auch ein kontinuierliches Verfahren angewandt werden.

Die Vorteile des Verfahrens der vorliegenden Erfindung gegenüber den Verfahren nach dem Stand der Technik liegen allgemein darin, dass in höherer Ausbeute ein Produkt mit dem gewünschten Isomerengehalt unter Verwendung wirtschaftlich vertretbarer Mengen eines Rhodium-Katalysators mit wirtschaftlich vernünftigen Geschwindigkeiten der Hydrierung hergestellt wird.

Die vorliegende Erfindung wird durch die folgenden Beispiele näher erläutert, ist jedoch nicht auf diese beschränkt. Hierin beziehen sich, sofern nicht anders vermerkt, sämtliche Angaben von Teilen und Prozentsätzen auf das Gewicht.

*Beispiele 1 bis 15*

In den nachstehenden Beispielen wurde nach

der folgenden allgemeinen Verfahrensweise gearbeitet.

Ein Autoklav wurde mit einem (5% Rhodium enthaltenden) Engelhard-Rhodium-auf-Aluminiumoxid-Katalysator in der angegebenen Menge, 200 Teilen n-Butylether und 200 Teilen Di-(4-aminophenyl)methan gefüllt und verschlossen. Der Autoklav wurde bei Raumtemperatur mit Wasserstoff bis zu einem Druck von etwa 275,8 bar (4000 psi) beschickt, und der Inhalt wurde auf die in Tabelle I angegebene Temperatur erhitzt. Der Verbrauch von Wasserstoff setzte bei 190 bis 196°C ein. Um ein Überschreiten der gewünschten Temperatur zu vermeiden, wurde Kühlung eingesetzt. Die Reaktion wurde nach Beendigung des Wasserstoff-Verbrauchs noch 30 bis 40 min auf der angegebenen Temperatur gehalten. Der Inhalt wurde dem Autoklaven bei Raumtemperatur entnommen und unter Vakuum filtriert. Im Gegensatz zu den Beispielen 1 bis 10 und 12 bis 15 wurde in Beispiel 11 der Druck während der Reaktionsdauer auf 275,8 bar (4000 psi) gehalten. Die Produkte wurden auf ihren Gehalt an dem Trans, transisomeren analysiert. Bei den bezeichneten Beispielen wurden die Ausbeuten bestimmt. Die erhaltenen Ergebnisse sind in Tabelle I aufgeführt.

*Tabelle I*

| Beispiel Nr. | Temperatur (°C) | Druck | | Katalysator | | $H_2$-Zeit (min) | Gehalt Trans,trans-isomer (%) | Ausbeute (mittels HPLC) (%) |
|---|---|---|---|---|---|---|---|---|
| | | (bar) | (psi) | Menge (Gew.-Teile) | Rh-Metall (Gew.-%) | | | |
| 1 | 200 | 275,8 | (4000) | 5 | 0,125 | 39 | 16,4 | 91 |
| 2 | 205 | 275,8 | (4000) | 5 | 0,125 | 30 | 18,5 | 84 |
| 3 | 210 | 275,8 | (4000) | 5 | 0,125 | 33 | 23,5 | — |
| 4 | 211 | 268,9 | (3900) | 5 | 0,125 | 30 | 16,4 | 52 |
| 5 | 216 | 275,8 | (4000) | 5 | 0,125 | 23 | 23,3 | 87 |
| 6 | 219 | 275,8 | (4000) | 5 | 0,125 | 18 | 23,4 | 85 |
| 7 | 222 | 275,8 | (4000) | 5 | 0,125 | 21 | 23,4 | 46 |
| 8 | 231 | 275,8 | (4000) | 5 | 0,125 | 12 | 36,4 | 80 |
| 9 | 211 | 275,8 | (4000) | 5 | 0,125 | 26 | 20,9 | 91 |
| 10 | 220 | 275,8 | (4000) | 5 | 0,125 | 28 | 27,7 | 91 |
| 11 | 230 | 275,8 | (4000) | 5 | 0,125 | 9 | 32 | 85 |
| 12 | 211 | 206,9 | (3000) | 5 | 0,125 | 36 | 21,9 | 81 |
| 13 | 211 | 137,9 | (2000) | 5 | 0,125 | 36 | 23,7 | 79 |
| 14 | 200 | 137,9 | (2000) | 1 | 0,025 | 60 | 18,8 | 73 |
| 15 | 200 | 275,8 | (4000) | 3 | 0,075 | 15 | 20,3 | 85 |

## Patentansprüche

1. Verfahren zur katalytischen Hydrierung von Di-(4-aminophenyl)methan zu flüssigem, 15 bis 40 Gew.-% des Trans,transisomeren enthaltenden Di-(4-aminocyclohexyl)methan, dadurch gekennzeichnet, dass Di-(4-aminophenyl)methan in Gegenwart eines Rhodiumträgerkatalysators unter einem Wasserstoffdruck von 137,9 bis 551,6 bar (2000 bis 8000 psi) und bei einer Temperatur von 196 bis 235°C hydriert wird.

2. Verfahren nach dem Anspruch 1, dadurch gekennzeichnet, dass die Temperatur von 200 bis 225°C beträgt.

3. Verfahren nach den Ansprüchen 1 und 2, dadurch gekennzeichnet, dass der Träger ausgewählt ist aus Kohlenstoff, Aluminiumoxid, Kieselgur, Bentonit, Asbest, Silicagel und Zirconiumoxid.

4. Verfahren nach den Ansprüchen 1 bis 3, dadurch gekennzeichnet, dass der Träger Aluminiumoxid ist.

5. Verfahren nach den Ansprüchen 1 bis 4, dadurch gekennzeichnet, dass die Reaktion in Gegenwart eines inerten Lösungsmittels durchgeführt wird.

6. Verfahren nach dem Anspruch 5, dadurch gekennzeichnet, dass das Lösungsmittel n-Butylether ist.

7. Verfahren nach dem Ansprüchen 1 bis 6, dadurch gekennzeichnet, dass der Katalysator in einer solchen Menge verwendet wird, dass die vorhandene Menge Rhodium mindestens 0,005 Gew.-%, bezogen auf die Menge des Di-(4-aminophenyl)-methans, beträgt.

8. Verfahren nach den Ansprüchen 1 bis 7, dadurch gekennzeichnet, dass der Katalysator in einer solchen Menge verwendet wird, dass die vorhandene Menge Rhodium etwa 0,01 bis 3 Gew.-% beträgt.

## Revendications

1. Procédé pour l'hydrogénation catalytique du di-(4-aminophényl)méthane en di-(4-aminocyclohexyl)méthane liquide contenant 15 à 40% en poids de l'isomère trans,trans, caractérisé en ce que l'on hydrogène le di-(4-aminophényl)-méthane en présence d'un catalyseur au rhodium sur support, sous une pression d'hydrogène de 137,9 à 551,6 bar et à une température de 196 à 235°C.

2. Procédé selon la revendication 1, caractérisé en ce que la température va de 200 à 225°C.

3. Procédé selon les revendications 1 et 2, caractérisé en ce que le support est choisi parmi le carbone, l'alumine, le kieselguhr, la bentonite, l'amiante, le gel de silice et l'oxyde de zirconium.

4. Procédé selon les revendications 1 à 3, caractérisé en ce que le support est l'alumine.

5. Procédé selon les revendications 1 à 4, caractérisé en ce que la réaction est effectuée en présence d'un solvant inerte.

6. Procédé selon la revendication 5, caractérisé en ce que le solvant est l'éther n-butylique.

7. Procédé selon les revendications 1 à 6, caractérisé en ce que l'on utilise le catalyseur en quantité telle que la quantité de rhodium présente soit d'au moins 0,005% en poids par rapport à la quantité du di-(4-aminophényl)méthane.

8. Procédé selon les revendications 1 à 7, caractérisé en ce que l'on utilise le catalyseur en quantité telle que la quantité de rhodium présente soit d'environ 0,01 à 3% en poids.

## Claims

1. Process for the catalytic hydrogenation of di-(4-aminophenyl)methane to give a liquid di-(4-aminocyclohexyl)methane containing 15 to 40% by weight of the trans,trans isomer, characterised in that di-(4-aminophenyl)methane is hydrogenated in the presence of a supported rhodium catalyst under a hydrogen pressure of 137.9 to 551.6 bar (2,000 to 8,000 psi) and at a temperature of 196 to 235°C.

2. Process according to Claim 1, characterised in that the temperature is 200 to 225°C.

3. Process according to Claims 1 and 2, characterised in that the support is selected from carbon, aluminium oxide, kieselguhr, bentonite, asbestos, silica gel and zirconium oxide.

4. Process according to Claims 1 to 3, characterised in that the support is aluminium oxide.

5. Process according to Claims 1 to 4, characterised in that the reaction is carried out in the presence of an inert solvent.

6. Process according to Claim 5, characterised in that the solvent is n-butyl ether.

7. Process according to Claims 1 to 6, characterised in that the catalyst is used in such a quantity that the amount of rhodium present is at least 0.005% by weight, based on the amount of di-(4-aminophenyl)methane.

8. Process according to Claims 1 to 7, characterised in that the catalyst is used in such an amount that the amount of rhodium present is about 0.01 to 3% by weight.